# EUROPEAN PATENT SPECIFICATION

(11) **EP 0 955 070 B1**
(45) Date of publication and mention of the grant of the patent: **03.03.2004**
(21) Application number: 97943129.3
(22) Date of filing: 01.10.1997
(51) Int. Cl.: A61M 5/158

(54) **MEDICAL ANESTHETIC NEEDLE**
MEDIZINISCHE NADEL FÜR DIE NARKOSE
AIGUILLE MEDICALE POUR ANESTHESIE

(43) Date of publication of application: 10.11.1999
(73) Proprietor: Dr. Japan Co. Ltd., Tokyo 160 (JP)
(72) Inventor: HIGUCHI, Akio, Dr. Japan Co., Ltd., Tokyo 160 (JP); HYUGAJI, Hayato, Dr. Japan Co., Ltd., Tokyo 160 (JP)
(74) Representative: Rost, Jürgen, Dipl.-Ing.
(86) International application number: PCT/JP1997/003497
(87) International publication number: WO 1999/016486

(56) References cited:
- EP-A- 0 150 281
- JP-A- 6 197 961
- JP-U- 3 018 360
- US-A- 4 565 545
- US-A- 5 350 393
- US-A- 5 533 977

## Description

### Technical Field

The present invention relates to a medical anesthetic needle including an inner needle, having a pencil-pointed tip, and a tubular outer needle, having the inner needle inserted thereinto and therethrough.

### Background Technology

Conventional inner needles, having a pencil-pointed tip (4), of anesthetic needles, were inserted into and through a straight-tubular outer needle having its fore-end section formed in a tapered shape tapering off towards the end thereof, i.e., a hollow, truncated conical shape. The fore-end part of the outer needle was deburred, but relatively sharp, therefore resulting in patients often feeling pain when needled. An anesthetic needle for easing such pain is disclosed in Japanese Utility Model No.3018360 where the fore-end section of a tubular outer needle has the edge formed round on the right-angled cut fore-end face thereof.

With an anesthetic needle with the fore-end section of a tubular outer needle thereof having the edge formed round on the right-angled cut fore-end face, the patient's pain when needled was eased, in comparison with an anesthetic needle having a sharp-edged fore-end section of its tubular outer needle. However, the extent of its pain easing was not enough.

The cause of a patient feeling pain when needled, even though the fore-end part of the tubular outer needle is formed round, is a gap between the fore-end section of its outer needle and its inner needle. The difference between the outer diameter of the inner needle and the inner diameter of the outer needle is around 0.05 to 0.1 mm, depending on the needle size. Thus, a gap of a maximum width of 0.05 to 0.1 mm may be formed since the inner needle tends to be decentered relative to the outer needle when needling. When a patient's nervous tissue is nipped in this gap, the patient feels pain.

US-A-5 350 393 discloses a needle as in the preamble of claim 1.

### Disclosure of The Invention

An object of the present invention is to provide a medical anesthetic needle including an inner needle of a straight needle shape, having a pencil-pointed tip, and an outer needle of a straight tubular shape, having the inner needle inserted thereinto and therethrough, wherein the medical anesthetic needle is capable of easing enough a patient's pain when needled.

To accomplish the object, a medical anesthetic needle, according to claim 1, of the present invention includes an inner needle of a straight needle shape, having a pencil-pointed tip, and an outer needle of a straight tubular shape, having the inner needle inserted thereinto and therethrough, wherein the fore-end part of the outer needle is formed in a tapered shape tapering off towards the end thereof, i.e., a hollow, truncated conical shape. Furthermore, the foremost section of the fore-end part is formed as a foremost-end part so that the minimum inner diameter of the foremost-end part is smaller than the outer diameter of a section of the inner needle excluding the tip, i.e., a main body of the inner needle.

The foremost-end part of the outer needle may be applied only with deburring so that the edge of its right-angled cut fore-end face is left sharp. If required, the foremost-end part may have the edge of its right-angled cut fore-end face worked to be round.

With an anesthetic needle of the present invention, when a patient is needled, the foremost-end part of its outer needle is located on the circumferential portion of a surface of either a section near the root of the tip or the main body of the inner needle where the outer diameter of the inner needle is equal to or greater than the minimum inner diameter of the foremost-end part of the outer needle. Thus, no gaps are formed at all between the foremost-end part of the outer needle and the inner needle. Therefore, the patient is not likely at all to feel pain caused by the patient's nervous tissue being nipped between the outer and inner needles.

### Brief Description of The Drawings

FIG. 1 is a plan view showing an external appearance of a medical anesthetic needle of an embodiment of the present invention,
FIG. 2 is a longitudinal sectional view showing the fore-end section of the medical anesthetic needle of FIG. 1, as enlarged,
FIG. 3 is a view showing a working process for the fore-end section of the outer needle of FIG. 2, and
FIG. 4 is a view corresponding to FIG. 2, of another embodiment.

### Best Embodiments of The Invention

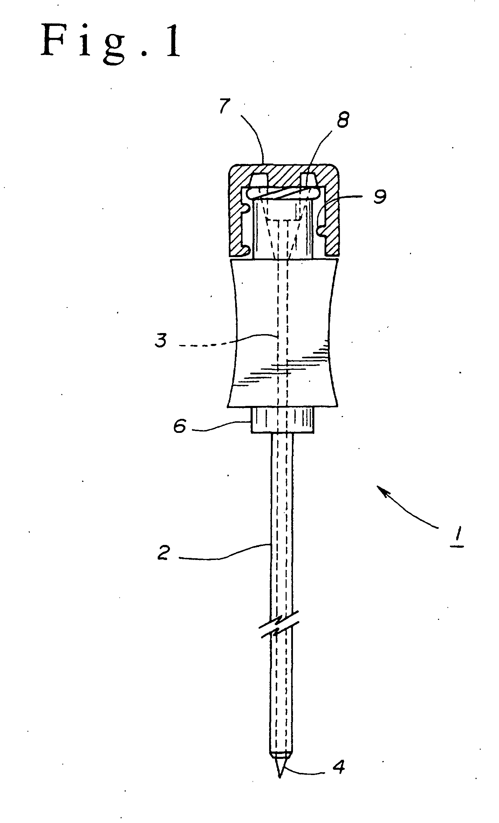

As shown in FIG. 1, an anesthetic needle 1 includes a hollow outer needle 2 made of stainless steel tube and a solid inner needle 3 made of stainless steel bar. The rear end of the outer needle 2 is fixed to a needle base 6. The inner needle 3 is inserted through a needle hole of the needle base 6 into the outer needle 2. A tip 4 of the inner needle 3 that protrudes from the fore end of the outer needle 2 is of a pencil-pointed shape, i.e., a conical shape. A cap 7 is fixed to the rear end of the inner needle 3. where a spiral-shaped female screw part 9 is formed on the internal circumferential surface of the cup 7. A spiral-shaped male screw part 8 that engages with the female screw part 9 of the cap 7, is disposed on a section protruding from the rear end of the needle base 6. When inserting the anesthetic needle, the male screw part 8 is screwed into the female screw part 9 to the limit, to define the axial limit position of the inner needle 3 relative to the outer needle 2.

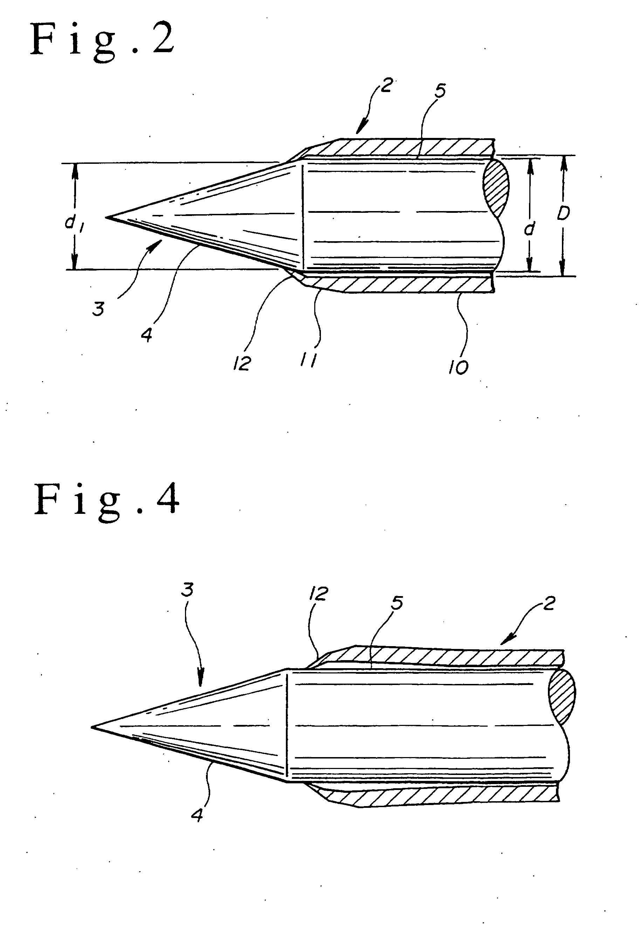

As shown in FIG. 2, the inner needle 3 includes a main body 5 of a round-column shape having a diameter d, and the tip 4 of the conical shape protruding axially from the main body 5 towards the fore-end side thereof. The outer needle 2 includes a main body 10 of a round cylindrical shape having an inner diameter D slightly greater than the diameter d of the main body 5 of the inner needle, a fore-end part 11 of a tapered shape tapering off and extending towards the fore-end side thereof, i.e., a hollow, truncated conical shape, and a foremost-end part 12 of a tapered shape further tapering off and extending from the fore-end part 11 towards the fore-end side thereof, i.e., a hollow, truncated conical shape. While the inner diameter of the fore-end part 11 is constant and equal to the inner diameter of the main body 10, the inner diameter of the foremost-end part 12 is gradually reduced along the axis thereof towards the fore-end side thereof with the minimum inner diameter thereof being slightly smaller than the outer diameter d of the main body 5 of the inner needle. While the outer diameter of the fore-end part 11 is equal to the outer diameter of the main body 10 at the joining section thereof with the main body 10, the outer diameter is continuously reduced from that section towards the fore-end side. While the outer diameter of the foremost-end part 12 is equal to the outer diameter of the fore-end part 11 at the joining section thereof with the fore-end part 11, the outer diameter is continuously reduced from that section towards the fore-end side, and is equal to the inner diameter at the foremost-end. The axial length of the foremost-end part 12 is smaller the axial length of the fore-end part 11. The inclination angle of the internal circumferential conical surface of the foremost-end part 12 is equal to or slightly greater than the inclination angle of the conical surface of the tip 4 of the inner needle 3.

FIGS. 1 and 2 show a state of the female screw part 9 of the cap 7 being screwed onto the male screw part 8 of the needle base 6 to the limit, i.e., the state of the anesthetic needle 1 when needling, where the foremost-end part 12 is located on the circumferential surface having an outer diameter d₁, of the tip 4 of the inner needle 3. Although the outer diameter d₁ is smaller than the outer diameter d of the main body 5 of the inner needle, the outer diameter d₁ is equal to or greater than the minimum inner diameter D₁, which will be described later, of the foremost-end part 12 of the outer needle. Therefore, the circumferential fore-end edge of the foremost-end part 12, having an inner diameter equal to the minimum inner diameter, comes in close contact with the entire circumferential surface near the root of the tip 4, having the diameter d₁, with circumferential fore-end edge either being kept as it is or elastically expanded. As a result, no gaps are formed between the circumferential surface of the tip 4 and the foremost-end part 12.

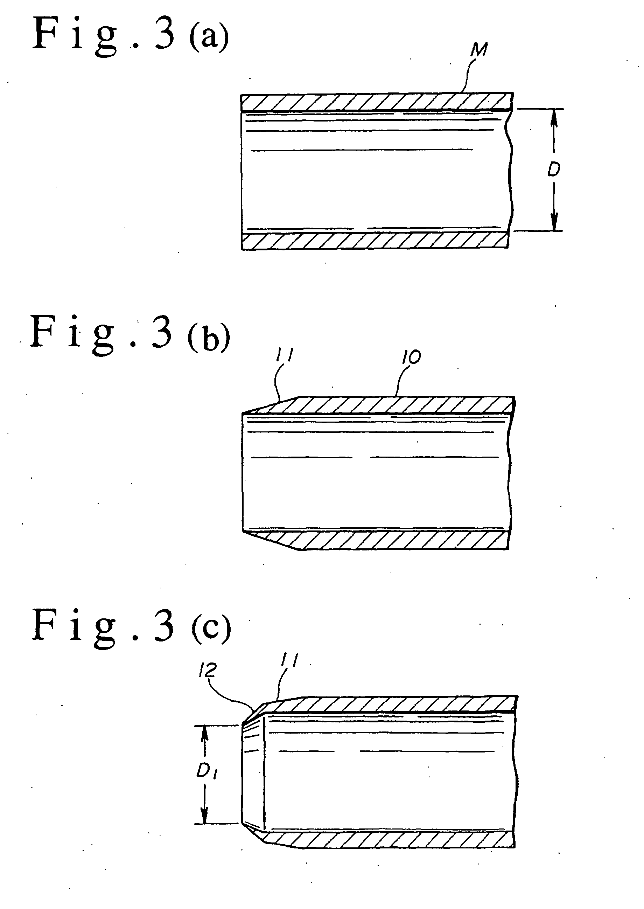

FIG. 3 is a view showing a process of forming the fore-end part 11 and the foremost-end part 12 of the outer needle 2. The corner of fore-end part of a stainless steel tube M having an inner diameter D, as shown in FIG. 3(a), is machine-cut to form a main body 10 of a hollow, round cylindrical shape and a sharp-edged fore-end part 11 of a hollow, truncated conical shape, as shown in FIG. 3(b). At this time, burr caused on the foremost end is removed by grinding. If required, the foremost end is finished so that the foremost end is round in a sectional plane containing the axis. Next, as shown in FIG. 3(c), the foremost-end section of the fore-end part 11 is roll-worked by squeezing so that the minimum inner diameter D₁ is obtained that is smaller than the outer diameter of the main body of the inner needle, thereby forming the foremost-end part 12 of a hollow truncated conical shape.

When a patient is needled with the anesthetic needle of FIG. 2, the internal circumferential fore-end edge of the foremost-end part 12 of the outer needle 2 comes in close contact with the circumferential surface, on the entire circumference thereof, of the conical-shaped tip 4 of the inner needle 3, and no gaps are thus formed between the foremost-end part 12 of the outer needle 2 and the tip 4 of the inner needle 3; therefore, the patient's nervous tissue is not likely at all to be nipped in a gap therebetween to cause any particular pain to the patient.

Although the anesthetic needle of FIG. 2 is arranged such that the foremost-end part 12 of the outer needle 2 is located on the tip 4 when the cap at the rear end of the inner needle is engaged with the needle base at the rear end of the outer needle, it also can be arranged such that the foremost-end part 12 of the outer needle 2 is located not on the tip 4 but on the main body 5, positioned behind the tip 4, of the inner needle 3 when the cap at the rear end of the inner needle is fully engaged to the limit with the needle base at the rear end of the outer needle so as to keep the inner needle from coming out of the outer needle.

With an anesthetic needle shown in FIG. 4, when needling, since the fore-end section of the outer needle 2 has its inner diameter expanded due to elastic deformation, and the circumferential fore-end edge of the foremost-end part 12 comes in close contact with the circumferential surface of the main body 5 of the inner needle 3, due to the resilience, no gaps are thus formed at all between the foremost-end part 12 and the inner needle 3. Therefore, similarly to the embodiment described earlier, the patient's nervous tissue is not likely to be nipped in a gap between the outer needle 2 and inner needle 3, whereby the patient cannot feel any particular pain by any means.

## Claims

1. A medical anesthetic needle (1) including an inner needle (3) of a straight needle shape, having a pencil-pointed tip (4), and an outer needle (2) the major portion of which has a straight tubular shape, having said inner needle inserted thereinto and therethrough from the rear end thereof, said medical anesthetic needle (1) **characterized in that**:
said tip (4), having the pencil-pointed shape, of said inner needle (3) extends axially from a main body (5) of a solid round-column shape towards the fore-end side;
said outer needle (2) includes:
a main body (10) of a hollow round-column shape having an inner diameter (D) greater than the outer diameter (d) of said main body (5) of said inner needle;
a fore-end part (11) of a hollow, truncated conical shape that extends axially from said main body (10) towards the fore-end side of said outer needle; and
a foremost-end part (12) of a hollow, truncated conical shape that further extends axially from said fore-end part towards the fore-end side of said outer needle;
wherein, while the inner diameter of said fore-end part (11) is constant and equal to the inner diameter of said main body (10), the inner diameter of said foremost-end part (12) is reduced along the axis thereof towards the fore-end thereof with the minimum inner diameter (D₁) of said foremost-end part (12) being smaller than or equal to the outer diameter (d) of said main body (5) of said inner needle.

2. A medical anesthetic needle according to claim 1, **characterized in that** the circumferential fore-end edge of said foremost-end part (12) is located on the circumferential portion of a surface of said tip (4) of said inner needle.

3. A medical anesthetic needle according to claim 1, **characterized in that** the circumferential fore-end edge of said foremost-end part (12) is located on the circumferential portion of a surface of said main body (5) of said inner needle.

## Patentansprüche

1. Eine medizinische anästhetische Nadel (1) umfassend eine innere Nadel (3) von gerader Nadelform, die eine Spitze (4) wie eine Bleistiftspitze aufweist, und eine äußere Nadel (2), deren Hauptteil eine gerade röhrenförmige Form besitzt, in und durch welche die innere Nadel von dem hinteren Ende her eingeführt ist, wobei die medizinische anästhetische Nadel (1) **dadurch gekennzeichnet ist, dass**:
die Spitze (4) der inneren Nadel (3), welche die Form einer Bleistiftspitze besitzt, erstreckt sich axial von einem Hauptkörper (5) einer massiven Rundzylinderform gegen das Vorder-Ende;
die äußere Nadel (2) umfasst:
eine Hauptkörper (10) einer hohlen Rundzylinderform, die einen inneren Durchmesser (D) besitzt, der größer als der äußere Durchmesser (d) des Hauptkörpers (5) der inneren Nadel ist;
ein Vorder-End-Teil (11) einer hohlen, gestutzten konischen Form, die sich axial von dem Hauptkörper (10) gegen die Vorder-End-Seite der äußeren Nadel erstreckt; und
ein vorderstes End-Teil (12) einer hohlen, gestutzten konischen Form, die sich weiter axial von dem Vorder-End-Teil gegen die Vorder-End-Seite der äußeren Nadel erstreckt;
wobei, während der innere Durchmesser des Vorder-End-Teils (11) konstant ist und gleich dem inneren Durchmesser des Hauptkörpers (10) ist, der innere Durchmesser des vordersten End-Teils (12) entlang seiner Achse gegen das Vorder-Ende reduziert ist, wobei der minimale innere Durchmesser (D₁) des vordersten End-Teils (12) kleiner oder gleich dein äußeren Durchmesser (d) des Hauptkörpers (5) der inneren Nadel ist.

2. Medizinische anästhetische Nadel gemäß Anspruch 1, **dadurch gekennzeichnet, dass** sich die umlaufende Vorder-End-Kante des vordersten End-Teils (12) auf dem umlaufenden Teil einer Oberfläche der Spitze (4) der inneren Nadel befindet.

3. Medizinische anästhetische Nadel gemäß Anspruch 1, **dadurch gekennzeichnet, dass** sich die umlaufende Vorder-End-Kante des vordersten End-Teils (12) auf dem umlaufenden Bereich einer Oberfläche des Hauptkörpers (5) der inneren Nadel befindet.

## Revendications

1. Aiguille médicale pour anesthésie (1) comprenant une aiguille interne (3) en forme d'aiguille droite, ayant une pointe semblable à celle d'une plume (4), et une aiguille externe (2), dont la plus grande partie est de forme tubulaire droite, dans et à travers laquelle est insérée l'aiguille interne à partir de l'extrémité arrière de l'aiguille externe, ladite aiguille médicale pour anesthésie (1) étant **caractérisée en ce que** :
ladite pointe (4), de forme semblable à celle d'une plume, de ladite aiguille interne (3) s'étend de façon axiale à partir d'un corps principal (5) de la forme d'une colonne circulaire pleine vers l'extrémité avant ;
ladite aiguille externe (2) comprend :
un corps principal (10) en forme de colonne circulaire creuse ayant un diamètre intérieur (D) supérieur au diamètre extérieur (d) dudit corps principal (5) de ladite aiguille interne ;
une extrémité avant (11) en forme de cône creux et tronqué qui s'étend axialement à partir dudit corps principal (10) vers l'extrémité avant de ladite aiguille externe ; et
une extrémité la plus avant (12) en forme de cône creux et tronqué qui s'étend plus loin de manière axiale à partir de ladite extrémité avant vers l'extrémité avant de ladite aiguille externe ;
dans laquelle, alors que le diamètre intérieur de ladite extrémité avant (11) est constant et égal au diamètre intérieur dudit corps principal (10), le diamètre intérieur de ladite extrémité la plus avant (12) diminue le long de l'axe de celle-ci vers l'extrémité avant de celle-ci, le diamètre intérieur minimal (D₁) de ladite extrémité la plus avant (12) étant inférieur ou égal au diamètre extérieur (d) dudit corps principal (5) de ladite aiguille interne.

2. Aiguille médicale pour anesthésie selon la revendication 1, **caractérisée en ce que** le bord circonférentiel d'extrémité avant de ladite extrémité la plus avant (12) est situé sur la partie circonférentielle d'une surface de ladite pointe (4) de ladite aiguille interne.

3. Aiguille médicale pour anesthésie selon la revendication 1, **caractérisée en ce que** le bord circonférentiel d'extrémité avant de ladite extrémité la plus avant (12) est situé sur la partie circonférentielle d'une surface dudit corps principal (5) de ladite aiguille interne.
